# EUROPÄISCHE PATENTSCHRIFT

(11) **EP 3 549 508 B1**
(45) Veröffentlichungstag und Bekanntmachung des Hinweises auf die Patenterteilung: **07.06.2023**
(21) Anmeldenummer: 18165501.0
(22) Anmeldetag: 03.04.2018
(51) Int. Cl.: A47L 15/44, A61L 9/02, A61L 9/04

(54) **DUFTSTOFFKÖRPER ZUR ABGABE VON DUFTSTOFFEN IN EINER GESCHIRRSPÜLMASCHINE**
FRAGRANCE BODY FOR RELEASING A FRAGRANCE IN A DISHWASHER
CORPS PARFUMÉ POUR LA DIFFUSION DE PARFUMS DANS UN LAVE-VAISSELLE

(43) Veröffentlichungstag der Anmeldung: 09.10.2019
(73) Patentinhaber: Mantz GmbH, 42651 Solingen (DE)
(72) Erfinder: Seefeld, Jörg, 42659 Solingen (DE)
(74) Vertreter: Kudla, Christof

(56) Entgegenhaltungen:
- EP-A2- 0 245 760
- WO-A1-96/38638
- WO-A1-97/12539
- WO-A1-2015/092684
- DE-A1- 10 036 850
- DE-A1- 10 237 066
- DE-A1-102005 004 487
- DE-B3-102006 003 286
- DE-U1- 8 315 008
- US-A1- 2005 148 479
- US-A1- 2005 167 522
- US-A1- 2014 076 983
- US-A1- 2018 193 511

## Beschreibung

Die vorliegende Erfindung betrifft einen Duftstoffkörper zur Abgabe von Duftstoffen, insbesondere in einer Geschirrspülmaschine, bestehend aus oder umfassend a) einem Trägerstoff bestehend aus oder umfassend mindestens ein Polymer und b) mindestens einen in dem Trägerstoff verteilten Duftstoff, wobei der Duftstoffkörper so ausgestaltet ist, dass er platzsparend und einfach am Anwendungsort befestigt werden kann. Die vorliegende Erfindung betrifft zusätzlich einen verpackten Duftstoffkörper, ein Verfahren zur Desodorierung und Beduftung von Räumen sowie die Verwendung von Duftstoffkörpern zur Desodorierung und Beduftung von Räumen.

In geschlossenen Geschirrspülmaschinen ohne ausreichende Frischluftzirkulation können unangenehme Gerüche auftreten, insbesondere wenn bereits benutztes und entsprechend verschmutztes Geschirr über einen längeren Zeitraum vor dem nächsten Spülgang verbleibt. Um solche unangenehmen Gerüche zu verringern oder zu beseitigen werden in Spülmaschinen sogenannte Geschirrspülmaschinendeos eingesetzt. Solche Geschirrspülmaschinendeos können auf unterschiedliche Arten ausgestaltet sein.

In der DE 203 21 180 U1 wird ein Duftabgabesystem mit einem Behälter und einer Vielzahl von im Aufnahmeraum des Behälters aufgenommenen Partikel zur Desodorierung von Geschirrspülmaschinen beschreiben. Die Partikel enthalten ein Trägermaterial sowie wenigstens einen Duftstoff und sind so ausgestaltet, dass sie den Duftstoff abgeben können. Um den Behälter mit den Partikeln in der Geschirrspülmaschine zu befestigen eine Aufhängeeinrichtung, bei der es sich um einen kleinen Haken handelt.

In der WO 91/00744 wird ein duftender Artikel beschrieben, der aus einem Polymer und einem in dem Polymer homogen verteilten Duftstoff besteht. Der Artikel weist eine Aufhängeeinrichtung auf, bei der es sich um eine Lasche handelt, mit der der Artikel in der Geschirrspülmaschine befestigt werden kann.

Die im Stand der Technik beschriebenen Geschirrspülmaschinendeos haben alle den Nachteil, dass sie in den Innenraum der Geschirrspülmaschine gehängt werden müssen. Hierdurch sind sie allerdings störend bei der Beladung und Entladung der Geschirrspülmaschine. Um einen Übergang des Duftstoffes auf das zu reinigende Geschirr zu vermeiden, sollte das Geschirr auch nicht in direkter Nachbarschaft zu den Spülmaschinendeos aufgestellt werden bzw. diese berühren. Daher nehmen die Spülmaschinendeos auch einen gewissen Platz in der Geschirrspülmaschine ein, der nicht für die Reinigung von Geschirr verwendet werden kann. Sofern die Spülmaschinendeos falsch angebracht werden, können sie die Funktionsweise der Geschirrspülmaschine beeinträchtigen, beispielsweise wenn sie so angebracht werden, dass sie die freie Rotation der Sprüharme beeinträchtigen.

Endverbraucher von Geschirrspülmaschinen hängen die Geschirrspülmaschinendeos häufig in den hinteren Bereich der Geschirrspülmaschine, um eine beeinträchtig beim Beladen bzw. Entladen der Spülmaschine durch die Geschirrspülmaschinendeos möglichst zu minimieren. Da die Geschirrspülmaschinendeos in diesem Fall allerdings außerhalb des täglichen Sichtbereichs des Verbrauchers angebracht sind, kommt es häufig dazu, dass diese Geschirrspülmaschinendeos vergessen werden und in der Geschirrspülmaschine verbleiben, obwohl sie keinen Geruch mehr abgeben. Auch wenn sich die bereits verbrauchten Geschirrspülmaschinendeos üblicherweise nicht nachteilig auf die Waschwirkung der Geschirrspülmaschine auswirken, so sind sie nicht mehr dazu geeignet um ihrer üblichen Bestimmung nachzukommen, nämlich unangenehmen Gerüche zu verringern oder zu beseitigen. Der Verbraucher wird erst wieder an den Wechsel des Geschirrspülmaschinendeos erinnert, wenn er einen unangenehmen Geruch der Geschirrspülmaschine war nimmt.

Weitere Beispiele eines im Stand der Technik bekannten Duftstoffkörpers finden sich in US 2014/0076983 A1, US2005/0167522 A1 und DE 83 15 008 U1.

Aufgabe der vorliegenden Erfindung war es daher einen Duftstoffkörper bereitzu stellen, der die oben beschriebenen Nachteile teilweise oder vollständig behebt. Insbesondere sollte der Duftstoffkörper so in der Spülmaschine anbringbar sein, dass er den regelmäßigen Belade- und Entladevorgang nicht stört, möglichst wenig Platz in der Geschirrspülmaschine einnimmt, die Funktion der Geschirrspülmaschine nicht beeinträchtigt und vom Verbraucher regelmäßig gesehen werden kann, um ihn an deinen Wechsel des Geschirrmaschinendeos zu erinnern.

Gelöst wird diese Aufgabe durch einen Duftstoffkörper (1) zur Abgabe von Duftstoffen, insbesondere in einer Geschirrspülmaschine, bestehend aus oder umfassend
a) einem Trägerstoff bestehend aus oder umfassend mindestens ein Polymer und
b) mindestens einen in dem Trägerstoff verteilten Duftstoff,
wobei der Duftstoffkörper (1) eine Mittelachse (M) aufweist und eine sich entlang der Mittelachse (M) oder parallel zur Mittelachse (M) erstreckende, durchlaufende längliche Vertiefung (N) zur Aufnahme eines länglichen Gegenstandes aufweist und wobei die Vertiefung (N) zumindest teilweise hinterschnitten ist.

Es hat sich überraschenderweise gezeigt, dass erfindungsgemäße Duftstoffkörper durch die längliche Vertiefung an länglichen Gegenständen, beispielsweise den Stangen eines Geschirrkorbes befestigt werden können. Erfindungsgemäße

Die WO 97/12539 A1 beschreibt einen Behälter zur Aufnahme einer Reinigungstablette, insbesondere zur Verwendung in einer Geschirrspülmaschine. Optional enthält die Reinigungstablette einen oder mehrere Duftstoffe. Der Behälter umfasst einen Hohlraum zur Aufnahme der Tablette sowie flüssigkeitsdurchlässige Wandungen. Weiterhin weist der Behälter auch eine Befestigungseinrichtung auf, mittels derer er z.B. am Gestänge einer Geschirrspülmaschine angeordnet werden kann.

Die DE 10 2005 004 487 A1 beschreibt einen Behälter für Reinigungsmittel zur Anordnung in einer Geschirrspülmaschine, der eine mit einem Schmelzverschluss verschlossene Abgabeöffnung aufweist. Der Schmelzverschluss schmilzt bei einer vorgegebenen Temperatur, wodurch in dem Behälter enthaltenes Reinigungsmittel durch die Abgabeöffnung freigesetzt wird. Weiterhin weist der Behälter Mittel zum Aufhängen, beispielsweise Haken auf, mittels derer er z.B. an einer Stange innerhalb der Geschirrspülmaschine befestigt werden kann.

Die DE 100 36 850 A1 beschreibt eine Vorrichtung zur Abgabe eines flüssigen Produktes in den Innenraum einer Geschirrspül- oder Waschmaschine mittels eines Dosierelements, wobei das flüssige Produkt mindestens einen dufterzeugenden und/oder geruchsabsorbierenden und/oder antimikrobiell wirkenden Wirkstoff enthält. Optional ist an dem Behältnis ein Befestigungselement vorgesehen. Die Abgabe des flüssigen Produktes über das Dosierelement erfolgt bevorzugt als Sprühstrahl, der insbesondere durch Druckausübung auf das Produktbehältnis aktiviert wird.

Die WO 96/38638 A1 beschreibt ein Spülmaschinen-Deodorant, das einen Feststoffpressling in einem mit einem Aufhänger versehenen Behältnis umfasst. Das Behältnis ist dabei aus Kunststoff, vorzugsweise aus Polypropylen. Der Pressling, in dem der Duftstoff enthalten ist, ist vorzugsweise auf Wachs-Basis.

Die DE 10 2006 003 286 B3 beschreibt ein Duftabgabesystem für Geschirrspülmaschinen mit einem schirmartigen Griffelement, das aufrecht in einem Besteckkorb angeordnet werden kann, wobei es teilweise aus dem Besteckkorb herausragt. Das Duftabgabesystem umfasst einen Behälter mit Öffnungen in der Mantelfläche, der mit einem Duftstoff oder einer duftstoffemittierenden Trägersubstanz befüllt ist. Die Trägersubstanz ist vorzugsweise ein Polymer.

Duftstoffkörper können durch die längliche Vertiefung auf längliche Gegenstände gesteckt werden und verbleiben aufgrund der zumindest teilweisen Hinterschneidung der Vertiefung auf dem länglichen Gegenstand ohne abzufallen.

Bei der länglichen Vertiefung kann es sich beispielsweise um eine Nut, eine Nut mit ein- oder beidseitiger Hohlkehle, eine Rundnut oder einen Zinken handeln.

Bei der Mittelachse (M) handelt es sich im Rahmen der vorliegenden Erfindung um eine gedachte Achse, die den Duftstoffkörper (1) mittig durchtritt.

Die Vertiefung ist zumindest teilweise hinterschnitten, sodass sie an zumindest einer Stelle eine Verengung aufweist. Diese Verengung bzw. Hinterschneidung führt dazu, dass ein länglicher Gegenstand in die Vertiefung gedrückt werden kann und es dabei zu einer kurzzeitigen Verformung des Duftstoffkörpers kommt und der Duftstoffkörper anschließend nicht von dem länglichen Gegenstand entnommen werden kann, ohne dass erneut eine ausreichende Kraft aufgewendet werden muss. Diese Art der Verbindung wird üblicherweise auch als Schnappverbindung bezeichnet. Erfindungsgemäß bevorzugt ist es, wenn der Duftstoffkörper mindestens eine oder drei Hinterschneidung aufweist.

Überraschenderweise hat es sich zudem gezeigt, dass erfindungsgemäße Duftstoffkörper nicht nur ausgezeichnet für den Einsatz in Geschirrspülmaschinen geeignet sind, sondern auch zur Desodorierung und Beduftung von anderen Räumen ausgesprochen gut geeignet sind. Dabei hat es sich insbesondere gezeigt, dass erfindungsgemäße Duftstoffkörper ausgezeichnet zur Desodorierung und Beduftung von Geschirrspülmaschinen, Kleiderschränken, Schubladen, Autos, Kühlschränken, Mülleimern, Taschen, insbesondere Sporttaschen oder Handtaschen, Textilwaschmaschinen oder Trocknern verwendet werden kann.

So ist es beispielsweise möglich den erfindungsgemäßen Duftstoffkörper der Kleidung während der Wäsche in einer Textilwaschmaschine zugegeben werden kann, anschließend kann die Wäsche samt Duftstoffkörper in den Trockner zum Trocknen der Textilien überführt werden. Nach erfolgtem Trocknen der Kleidung kann der Duftstoffkörper mit seiner länglichen Vertiefung an das Gestänge von Kleiderbügeln angebracht werden. Hierdurch wird eine Desodorierung der Wäsche während des Textilwäsche und des Trocknungsvorgangs erreicht und anschließend verbleibt der Duftstoffkörper in der Nähe der Kleidung und verhindert ein abnehmen des Duftes. Der Anwender hat somit ab der ersten Wäsche bis zum Tragen der Kleidung ein angenehmes Frischegefühl. Natürlich besteht auch die Möglichkeit, den erfindungsgemäßen Duftstoffkörper lediglich der Wäsche in der Textilwaschmaschine oder lediglich dem Trockner beizugeben oder lediglich am Kleiderbügel zu verwenden.

Bei der Verwendung des erfindungsgemäßen Duftstoffkörpers in Autos kann der Duftstoffkörper beispielsweise mit seiner länglichen Vertiefung an die Lüftungsschlitze der Autolüftungsanlage befestigt werden. Dieses Befestigen kann dabei direkt erfolgen oder über eine zusätzliche Halterung erfolgen. Bei einer zusätzlichen Halterung wird der Duftstoffköper in eine Halterung eingeführt, die so ausgebildet ist, dass sie in die längliche Vertiefung des Duftstoffkörpers formschlüssig eingeführt werden kann und gleichzeitig Befestigungsmöglichkeiten zur Befestigung an den Lüftungsschlitzen der Autolüftungsanlage aufweist. Durch die Verwendung einer entsprechenden Halterung wird ein direkter Kontakt zwischen dem Duftstoffkörper und den die Lüftungsschlitze, die meistens aus Kunststoff bestehen, vermieden.

Bei der Verwendung des erfindungsgemäßen Duftstoffkörpers in Kühlschränken kann der Duftstoffkörper beispielsweise mit der länglichen Vertiefung an das Gitter der Ablageflächen angebracht werden.

Es ist erfindungsgemäß bevorzugt, wenn der Duftstoffkörper im Wesentlichen eine längliche Form mit einer Längsachse aufweist und die Längsachse vorzugsweise der Mittelachse entspricht.

Insbesondere bei einer länglichen Form des Duftstoffkörpers kann der Duftstoffkörper platzsparend angebracht werden, da er aufgrund seiner länglichen Vertiefung auf längliche Gegenstände gesteckt werden kann und somit kaum Platz einnimmt. Bei dem länglichen Gegenstand kann es sich beispielsweise um ein Rohr oder einen Rundstab handelt, beispielsweise ein Rundstab eines Geschirrspülmaschinenkorbes.

Erfindungsgemäß bevorzugt weist der Duftstoffkörper im Wesentlichen die Form eines Würfels, einer Kugel, eines geraden Prismas oder eines Zylinders auf. Bei dem geraden Prisma kann es sich beispielsweise um ein Prisma mit einem Viereck (Quader), Quadrat (quadratisches gerades Prisma), Dreieck, Fünfeck, Sechseck oder Vieleck als Grundfläche handeln.

Bei der Längsachse (L) handelt es sich im Rahmen der vorliegenden Erfindung um eine gedachte Achse des Duftstoffkörpers, die der Richtung seiner größten Ausdehnung entspricht. Im Falle eines Zylinders oder eines geraden Prismas verläuft die Längsachse senkrecht zu den Grundflächen.

Eigene Untersuchungen haben gezeigt, dass erfindungsgemäße Duftstoffkörper besonders gute Eigenschaften aufweisen, wenn die Duftstoffkörper eine längliche Form aufweisen und dabei eine maximale Länge entlang der Längsachse von 2 bis 15 cm aufweisen, vorzugsweise von 3 bis 10 cm aufweisen, besonders bevorzugt von 4 bis 8 cm aufweisen. Für den Einsatz in Geschirrspülmaschinen hat sich eine Länge von 6 cm ± 1 cm als besonders bevorzugt herausgestellt.

Eine maximale Dicke des Duftstoffkörpers senkrecht zur Längsachse von kleiner gleich 6 cm, vorzugsweise von kleiner gleich 4 cm, besonders bevorzugt von kleiner gleich 3 cm hat sich ebenfalls als besonders bevorzugt herausgestellt. Im Fall eines Zylinders entspricht die maximale Dicke des Duftstoffkörpers dem Durchmesser. Eine minimale Dicke des Duftstoffkörpers senkrecht zur Längsachse von größer gleich 0,5 cm, vorzugsweise von größer gleich 1 cm, besonders bevorzugt von größer gleich 1,5 cm hat sich ebenfalls als besonders bevorzugt herausgestellt.

Für den Einsatz in Geschirrspülmaschinen hat sich eine Dicke des Duftstoffkörpers senkrecht zur Längsachse von 2 cm ± 0,5 cm als besonders bevorzugt herausgestellt.

In einer bevorzugten Ausgestaltung des erfindungsgemäßen Duftstoffkörpers weist der Duftstoffkörper eine oder mehrere Ausnehmungen auf. Eine Ausnehmung kann beispielsweise durch eine Mulde, einen Schlitz, eine Kerbe, ein Sackloch oder ein durchgängiges Loch gebildet werden. Ausnehmungen haben die Funktion die Oberfläche des Duftstoffkörpers zu erweitern, ohne die Gesamtgrö-βe des Duftstoffkörpers zu erhöhen. Hierdurch wird die Diffusion des Duftstoffes an die Oberfläche des Duftstoffkörpers während des Gebrauches des Duftstoffkörpers vereinfacht.

In einer Ausgestaltung der vorliegenden Erfindung ist es möglich, dass verschiedene Ausnehmungen miteinander kombiniert werden, so kann ein Duftstoffkörper beispielsweise eine Kerbe, eine Mulde und ein durchgehendes Loch oder jede andere beliebige mögliche Kombination von verschiedenen Ausnehmungen aufweisen.

Erfindungsgemäß bevorzugt ist es allerdings, wenn jeweils eine Sorte von Ausnehmungen miteinander kombiniert werden, so kann der Duftstoffkörper beispielsweise eine Reihe von Schlitzen aufweisen, die vorzugsweise parallel zueinander angeordnet sind.

Falls der Duftstoffkörper beispielsweise eine längliche Form aufweist, kann die Ausnehmung bzw. können die Ausnehmungen im Wesentlichen entlang der Längsachse des Duftstoffkörpers verlaufen oder im Wesentlichen quer zu seiner Längsachse verlaufen und den Duftstoffkörper teilweise oder gegebenenfalls vollständig durchtreten. Selbstverständlich kann die Ausnehmung bzw. können die Ausnehmungen auch in jedem beliebigen anderen Winkel zur Längsachse vorgesehen sein.

Dabei ist es erfindungsgemäß bevorzugt, wenn die Ausnehmungen im Wesentlichen die gleiche Dicke aufweisen, wie das zwischen verschiedenen Ausnehmungen verbleibende Material der Duftstoffkörper. Es ist erfindungsgemäß besonders bevorzugt, wenn die alternierend vorliegenden Ausnehmungen und das verbleibende Material überwiegend eine Materialstärke von 2 mm ±1 mm (vorzugsweise 2 mm ±0,5 mm) aufweisen und/oder wenn die Materialstärke im gesamten Duftstoffkörper überwiegend maximal 2 mm ±1 mm (vorzugsweise 2 mm ±0,5 mm) aufweist.

Eigene Untersuchungen haben überraschender Weise ergeben, dass bei einer Materialstärke von 2 mm ±1 mm (vorzugsweise 2 mm ±0,5 mm) eine optimale Diffusion des Duftstoffes innerhalb der Lebensdauer des Duftstoffkörpers des Duftstoffes durch den Trägerstoff erfolgen kann und dass hierdurch immer ausreichend Duftstoff an der Oberfläche des Duftstoffkörpers zur Verfügung steht. Bei Materialstärken von über 3 mm benötigt die Diffusion des Duftstoffes verhältnismäßig mehr Zeit, sodass der Duftstoffkörper vom Konsumenten bereits als nicht mehr duftend beurteilt wird, obwohl im Inneren des Duftstoffkörpers noch ausreichend Duftstoff vorhanden ist.

Als Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, oc-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang- Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die allgemeine Beschreibung der einsetzbaren Duftstoffe stellt dabei allgemein die unterschiedlichen Substanzklassen von Riechstoffen dar. Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 g/mol, während Molmassen von 300 g/mol und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Durch eine geeignete Auswahl der genannten Duftstoffe bzw. Parfümöle kann auf diese Weise für erfindungsgemäße Mittel sowohl der Produktgeruch unmittelbar beim Öffnen des fabrikneuen Duftkörpers als auch der Gebrauchsduft, beispielsweise beim Einsatz in einer Geschirrspülmaschine, beeinflusst werden. Diese Dufteindrücke können selbstverständlich gleich sein, können sich aber auch unterscheiden. Für den letzteren Geruchseindruck ist die Verwendung haftfesterer Riechstoffe vorteilhaft, während zur Produktbeduftung auch leichterflüchtige Riechstoffe einsetzbar sind. Haftfeste Riechstoffe, die im Rahmen der vorliegenden Erfindung einsetzbar sind, sind beispielsweise die ätherischen Öle wie Angelikawurzelöl, Anisöl, Amikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Stemanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, -Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzim talkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p- Methylchinolin, Methyl-β-naphthylketon, Methyl-n-nonylacetaldehyd, Methyl-n- nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral und Zitronellal.

Ein erfindungsgemäßer Duftstoffkörper ist bevorzugt, wobei der Duftstoffköper zusätzlich c) Füllstoffe umfasst, vorzugsweise Füllstoffe ausgewählt aus der Liste umfassend Calciumcarbonat, Glasfasern, Glaskugeln, Kohlefasern, natürliches oder kalziniertes Kaolin, Ruß, Silikate, amorphes oder kristallines SiO₂, Talk, Kreide, Aluminiumhydroxid, Magnesiumhydroxid, Aluminiumnitrit, Aluminiumsilikat, Bariumsulfat, Calciumsulfat, Kieselsäure, Quarzmehl, Aerosil, Kieselsäuren, Tonerde und Wollastonit.

Besonders bevorzugt sind dabei Füllstoffe, die eine BET-Oberfläche von mehr als 200 m²/g aufweisen. Es hat sich gezeigt, dass diese Füllstoffe in der Lage sind, Duftstoffe aufzunehmen und während der Verwendung des Duftstoffkörpers langsam abzugeben. Durch die Verwendung von Füllstoffen mit einer BET-Oberfläche von mehr als 200 m²/g ist es daher möglich, die Lebensdauer (beispielsweise die Anzahl der Waschzyklen) der erfindungsgemäßen Duftstoffkörper zu erhöhen, da die aufgenommenen Duftstoffe langsam und konstant abgegeben werden können.

Ein erfindungsgemäßer Duftstoffkörper ist ebenfalls bevorzugt, wobei der Duftstoffkörper zusätzlich d) Farbstoffe und/oder Pigmente umfasst. Dabei ist es erfindungsgemäß bevorzugt, wenn die Farbstoffe so gewählt sind, dass der Duftstoffkörper eine Signalwirkung hervorruft, d. h. der Duftstoffkörper beispielsweise rot, gelb, orange, neongelb, neonorange oder neonrot gefärbt ist. Entsprechend ist es erfindungsgemäß bevorzugt, wenn die d) Farbstoffe und/oder Pigmente eine Signalfarbe aufweisen, vorzugsweise rot, gelb, orange, neongelb, neonorange oder neonrot.

Durch das Einfärben des Duftstoffkörpers in Signalfarben ist der Duftstoffkörper für den Endverbrauch während der Verwendung immer gut sichtbar und die Gefahr, dass der Endverbraucher vergisst den Duftstoffkörper auszutauschen wird minimiert. Natürlich ist auch das Einfärben des Duftstoffkörpers in allen anderen Farben möglich.

In einer alternativen Ausgestaltung des erfindungsgemäßen Duftstoffkörpers sind die d) Farbstoffe und/oder Pigmente so gewählt, dass sie sich während der Benutzung in einer Geschirrspülmaschine auswaschen bzw. verbleichen. Hierdurch ist für den Anwender erkennbar, wenn der Duftstoffkörper eine gewisse Anzahl an Waschdurchgängen durchlaufen hat und ausgetauscht werden muss. Dabei sind die Duftstoffkonzentration und der Farbstoff bzw. das Pigment so aufeinander abgestimmt, dass der Verbrauch des Duftstoffes idealerweise gleichzeitig mit der Entfärbung des Duftstoffkörpers erfolgt. Entsprechende Farbstoffe bzw. Pigmente, die während der Benutzung auswaschbar sind bzw. verbleichen sind dem Fachmann bekannt und er kann diese Farbstoffe bzw. Pigmente entsprechend wählen.

In einer erfindungsgemäß bevorzugten Ausgestaltung umfasst der Duftstoffkörper zusätzlich e) weitere Additive, insbesondere antimikrobiellen Wirkstoffe, Germizide, Fungizide, Antioxidantien oder Korrosionsinhibitoren.

Unter dem Begriff der "Polymer" werden im Rahmen der vorliegenden Anmeldung Polymerisate, Polyaddukte und Polykondensate zusammengefasst.

Ein erfindungsgemäßer Duftstoffkörper ist besonders bevorzugt, wobei das Polyethylen ein verzweigtes Polyethylen mit einer Dichte von kleiner 0,95 g/cm³ ist.

In der Ausgestaltung des erfindungsgemäßen Duftstoffkörpers besteht oder umfasst der Trägerstoff Ethylen-Vinylacetat-Copolymer und Polyethylen, vorzugsweise Polyethylen mit einer Dichte von kleiner 0,95 g/cm³.

Eigene Untersuchungen haben gezeigt, dass Ethylen-Vinylacetat-Copolymer besonders gute Eigenschaften bei der Aufnahme von Duftstoffen aufweist allerdings bei der Verwendung in Geschirrspülmaschinen häufig nicht ausreichend hohe mechanische Eigenschaften zeigt, da das Ethylen-Vinylacetat-Copolymer zu weich ist. Durch die Zugabe von Polyethylen kann allerdings die mechanische Beständigkeit des Ethylen-Vinylacetat-Copolymers signifikant verbessert werden, ohne dass die gewünschten Eigenschaften nachteilig beeinflusst werden.

Eigene Untersuchungen haben gezeigt, dass es bei der Verwendung von einer Mischung aus Ethylen-Vinylacetat-Copolymer und Polyethylen besonders bevorzugt ist, wenn die verwendeten Duftstoffe eine geringe Polarität aufweisen, d.h., möglichst unpolar sind. Je unpolarer die ausgewählten Duftstoffe sind, desto mehr Duftstoff kann von dem Ethylen-Vinylacetat-Copolymer bzw. der Mischung aus Ethylen-Vinylacetat-Copolymer und Polyethylen aufgenommen werden.

In einer alternativen Ausgestaltung, welche nicht Gegenstand der vorliegenden Erfindung ist, wird ein Duftstoffkörper bereitgestellt, wobei das Polymer oder zwei, drei, mehr als drei oder sämtliche Polymere Celluloseester sind, vorzugsweise Cellulosester ausgewählt aus der Gruppe umfassend, Celluloseacetat, Celluloseacetatphthalat, Celluloseacetatbutyrat, Celluloseacetatpropionat, Cellulosebutyrat und Cellulosepropionat.

Ein erfindungsgemäßer Duftstoffkörper ist besonders bevorzugt, wobei das Polymer bzw. die Polymere nicht wasserlöslich ist bzw. sind.

Ein erfindungsgemäßer Duftstoffkörper ist besonders bevorzugt, wobei es sich bei dem Polymer oder bei einem, zwei, drei, mehr als drei oder sämtlichen Polymeren um einen thermoplastischen Kunststoff handelt.

Ein erfindungsgemäßer Duftstoffkörper ist besonders bevorzugt, wobei das Polymer oder ein, zwei, drei, mehr als drei oder sämtliche Polymer einen Schmelzpunkt von über 100 °C, vorzugsweise von über 120 °C, besonders bevorzugt von über 130 °C aufweist.

Ein erfindungsgemäßer Duftstoffkörper ist besonders bevorzugt, wobei der Duftstoffkörper
95 bis 60 Gew.-%, vorzugsweise 90 bis 70 Gew.-% Trägerstoff
5 bis 40 Gew.-%, vorzugsweise 10 bis 30 Gew.-% Duftstoff,
und
0 bis 20 Gew.-%, vorzugsweise 0 bis 10 Gew.-% weitere Bestandteile umfasst oder daraus besteht, bezogen auf das Gesamtgewicht des Duftstoffkörpers.

In einer Ausgestaltung des erfindungsgemäßen Duftstoffkörpers weist der Trägerstoff eine poröse oder mikroporöse Beschaffenheit auf. Im Sinne dieser Erfindung ist ein Trägerstoff porös, wenn er Kanäle enthält, die es gestatten, dass Duftstoffe durch die Kanäle unmittelbar von der einen auf die entgegengesetzte Seite des Trägerstoffes gelangen können und die Kanäle einen Durchmesser von größer gleich 2 nm und kleiner gleich 50 µm aufweisen. Mikroporös ist ein Trägerstoff im Sinne dieser Erfindung, wenn die Kanäle einen Durchmesser von weniger als 2 nm aufweisen.

Ein Duftstoffkörper zur Abgabe von Duftstoffen, insbesondere in einer Geschirrspülmaschine, ist besonders bevorzugt bestehend aus oder umfassend
a) einem Trägerstoff bestehend aus oder umfassend eine Mischung aus Ethylen-Vinylacetat-Copolymer und Polyethylen und
b) mindestens einen in dem Trägerstoff verteilten Duftstoff,

wobei der Duftstoffkörper (1) eine Mittelachse (M) aufweist und eine sich entlang der Mittelachse (M) oder parallel zur Mittelachse (M) erstreckende, durchlaufende längliche Vertiefung (N) zur Aufnahme eines länglichen Gegenstandes aufweist und wobei die Vertiefung (N) zumindest teilweise hinterschnitten ist,
und wobei der Duftstoffkörper im Wesentlichen eine längliche Form mit einer Längsachse aufweist und die Längsachse vorzugsweise der Mittelachse entspricht,
und wobei der Duftstoffkörper eine maximale Länge entlang der Längsachse von 2 bis 15 cm aufweisen, vorzugsweise von 3 bis 10 cm aufweisen, besonders bevorzugt von 4 bis 8 cm aufweisen,
und wobei der Duftstoffkörper eine maximale Dicke senkrecht zur Längsachse von kleiner gleich 6 cm, vorzugsweise von kleiner gleich 4 cm, besonders bevorzugt von kleiner gleich 3 cm aufweist,
und wobei der Duftstoffkörper eine oder mehrere Ausnehmungen aufweist, vorzugsweise in Form einer Mulde, eines Schlitzes, einer Kerbe, eines Sacklochs oder eines durchgängigen Lochs.

Ein weiterer Aspekt der vorliegenden Erfindung betrifft einen verpackten erfindungsgemäßen Duftstoffkörper, wobei der Duftstoffkörper in einer luftdichten Verpackung angeordnet ist.

Ein verpackter Duftstoffkörper ist erfindungsgemäß bevorzugt, wobei der Duftstoffkörper in einem luftdichten Sachet angeordnet ist.

In einer bevorzugten Ausgestaltung ist das luftdichte Sachet wasserlöslich. In diesem Fall kann der Duftstoffkörper gemeinsam mit dem Sachet in einer Geschirrspülmaschine befestigt werden und das Sachet löst sich beim ersten Spülgang vollständig auf, sodass der Duftstoffkörper vom Endverbraucher nicht weiter ausgepackt werden muss.

Ein weiterer Aspekt der vorliegenden Erfindung betrifft eine Verwendung eines erfindungsgemäßen Duftstoffkörpers zur Desodorierung und Beduftung von Räumen, vorzugsweise von Innenraum von Gebäuden, Möbeln, Innenraum von Fahrzeugen oder technischen Geräten, vorzugsweise um einen Innenraum von Textilwaschmaschinen, Trocknern oder Geschirrspülmaschinen.

Ein weiterer Aspekt der vorliegenden Erfindung betrifft ein Verfahren zur Desodorierung und Beduftung von Räumen, wobei ein erfindungsgemäßer Duftstoffkörper in den Raum eingebracht wird.

Ein erfindungsgemäßes Verfahren ist bevorzugt, wobei der Duftstoffkörper zumindest zeitweise auf Temperaturen zwischen 30 und 95 °C erwärmt wird.

Ein erfindungsgemäßes Verfahren ist bevorzugt, wobei es sich bei dem Raum um einen Innenraum von Gebäuden, Möbeln, Innenraum von Fahrzeugen oder technischen Geräten, vorzugsweise um einen Innenraum von Textilwaschmaschinen, Trocknern oder Geschirrspülmaschinen handelt. Ganz besonders bevorzugt handelt es sich um den Innenraum einer Geschirrspülmaschine.

Ein weiterer Aspekt nicht Gegenstand der vorliegenden Erfindung betrifft ein Verfahren zur Herstellung von erfindungsgemäßen Duftstoffkörpern umfassend die folgenden Schritte:
- Herstellen oder Bereitstellen von Polymerpartikeln, vorzugsweise Polymerpartikeln aus Ethylen-Vinylacetat-Copolymer,
- Herstellen oder Bereitstellen eines Duftstoffes oder einer Duftstoffmischung,
- Imprägnieren der hergestellten oder bereitgestellten Polymerpartikel mit dem hergestellten oder bereitgestellten Duftstoff oder der Duftstoffmischung, vorzugsweise ohne einer Zuführung von Wärme,
- Optional Herstellen oder Bereitstellen von weiteren Polymerpartikeln, vorzugsweise Polymerpartikeln Polyethylen,
- Optionale Zugabe von weiteren Bestandteilen, vorzugsweise Bestandteile ausgewählt aus der Liste bestehend aus Füllstoffen, Farbstoffen, Pigmenten und weiteren Additiven, insbesondere antimikrobiellen Wirkstoffen, Germiziden, Fungiziden, Antioxidantien oder Korrosionsinhibitoren,
- Mischen aller Komponenten,
- Schmelzen der Komponenten,
- Herstellen eines Duftstoffkörpers mittels Extrusions- oder Spritzgußverfahren aus den geschmolzenen Komponenten.

Die Erfindung ist nachstehend anhand der Zeichnungen beispielhaft erläutert. Diese zeigen in:
Fig.1 einen erfindungsgemäßen Duftstoffkörper in einer perspektivischen Darstellung,
Fig. 2 eine Ansicht von unten des erfindungsgemäßen Duftstoffkörpers aus Fig. 1,
Fig. 3 eine Ansicht von der linken Seite des erfindungsgemäßen Duftstoffkörpers auf Fig. 1,
Fig. 4 einen erfindungsgemäßen Duftstoffkörper in einer perspektivischen Darstellung.

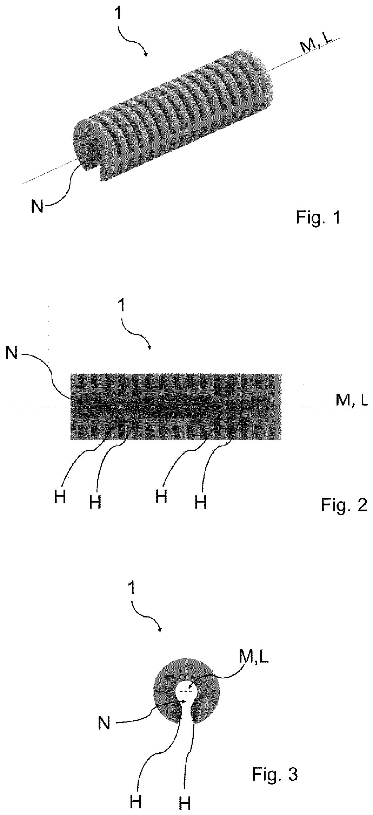

Figur 1 zeigt einen erfindungsgemäßen Duftstoffkörper (1) in einer perspektivischen Darstellung. Der Duftstoffkörper weist eine Mittelachse (M) auf, die aufgrund der zylindrischen (länglichen) Form des Duftstoffkörpers vorliegend auch der Längsachse (L) entspricht. Entlang der der Mittelachse (M) bzw. Längsachse (L) erstreckt sich eine durchlaufende längliche Vertiefung (N), die so ausgebildet ist, dass ein länglicher Gegenstand, beispielsweise ein Rohr oder eine Stange, in der länglichen Vertiefung aufgenommen werden kann. Der abgebildete Duftstoffkörper besteht aus einer Mischung aus Ethylen-Vinylacetat-Copolymer und Polyethylen als Trägerstoff und einer in dem Trägerstoff verteilten Duftstoffmischung. Der abgebildete Duftstoffkörper weist mehrere Ausnehmungen auf, bei denen es sich um Schlitze handelt, die alle parallel zueinander angeordnet sind und quer zur Längsachse (L) verlaufen. Der abgebildete Duftstoffkörper weist eine Länge entlang der Längsachse (L) von 6 cm ± 1 cm auf und eine Dicke (entspricht vorliegend dem Durchmesser des Zylinders) des Duftstoffkörpers senkrecht zur Längsachse von 2 cm ± 0,5 cm. Die Ausnehmungen habe eine Dicke von ca. 2 mm und die Materialstärke des zwischen den Ausnehmungen verbliebenen Materials beträgt ca. 2 mm.

Figur 2 zeigt eine Ansicht von unten des erfindungsgemäßen Duftstoffkörpers (1) aus Fig. 1. In dieser Ansicht ist die durchlaufende längliche Vertiefung (N) zur Aufnahme eines länglichen Gegenstandes gut zu erkennen, die an zwei Stellen Hinterschneidungen (H) aufweist.

Figur 3 zeigt eine Ansicht von der Seite des erfindungsgemäßen Duftstoffkörpers (1) aus Fig. 1. In dieser Ansicht ist die durchlaufende längliche Vertiefung (N) zur Aufnahme eines länglichen Gegenstandes gut zu erkennen, die an zwei Stellen Hinterschneidungen (H) aufweist. Die Hinterschneidungen (H) sind dabei so ausgebildet, dass Sie die Vertiefung (N) im Außenbereich verengen.

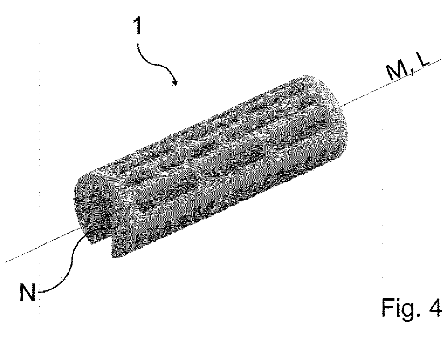

Fig. 4 zeigt einen erfindungsgemäßen Duftstoffkörper in einer perspektivischen Darstellung. Der dargestellte Duftstoffkörper (1) unterscheidet sich von dem in Figur dargestellten Duftstoffkörper (1) lediglich durch die Anordnung der Ausnehmungen, bei denen es sich im vorliegenden Fall um Schlitze handelt, die parallel zur Längsachse (L) angeordnet sind.

## Patentansprüche

1. Duftstoffkörper (1) zur Abgabe von Duftstoffen, insbesondere in einer Geschirrspülmaschine, bestehend aus oder umfassend
a) einem Trägerstoff bestehend aus oder umfassend mindestens ein Polymer und
b) mindestens einen in dem Trägerstoff verteilten Duftstoff,
wobei der Duftstoffkörper (1) eine Mittelachse (M) aufweist und eine sich entlang der Mittelachse (M) oder parallel zur Mittelachse (M) erstreckende, durchlaufende längliche Vertiefung (N) zur Aufnahme eines länglichen Gegenstandes aufweist und wobei die Vertiefung (N) zumindest teilweise hinterschnitten ist,
wobei der Duftstoffkörper durch die längliche Vertiefung (N) auf längliche Gegenstände gesteckt werden und aufgrund der zumindest teilweisen Hinterschneidung der Vertiefung auf dem länglichen Gegenstand verbleiben kann, ohne abzufallen,
**dadurch gekennzeichnet, dass** der Trägerstoff aus einer Mischung aus Ethylen-Vinylacetat-Copolymer und Polyethylen besteht oder diese Mischung umfasst.

2. Duftstoffkörper nach Anspruch 1, wobei der Duftstoffkörper im Wesentlichen eine längliche Form mit einer Längsachse aufweist und die Längsachse vorzugsweise der Mittelachse entspricht.

3. Duftstoffkörper nach einem der vorherigen Ansprüche, wobei der Duftstoffköper zusätzlich c) Füllstoffe umfasst, vorzugsweise Füllstoffe ausgewählt aus der Liste umfassend Calciumcarbonat, Glasfasem, Glaskugeln, Kohlefasern, natürliches oder kalziniertes Kaolin, Ruß, Silikate, amorphes oder kristallines SiO₂, Talk, Kreide, Aluminiumhydroxid, Magnesiumhydroxid, Aluminiumnitrit, Aluminiumsilikat, Bariumsulfat, Calciumsulfat, Kieselsäure, Quarzmehl, Aerosil, Kieselsäuren, Tonerde und Wollastonit.

4. Duftstoffkörper nach einem der vorherigen Ansprüche, wobei der Duftstoffkörper eine oder mehrere Ausnehmungen aufweist, vorzugsweise in Form einer Mulde, eines Schlitzes, einer Kerbe, eines Sacklochs oder eines durchgängigen Lochs.

5. Duftstoffkörper nach einem der vorherigen Ansprüche, wobei der Duftstoffkörper zusätzlich e) weitere Additive umfasst, insbesondere antimikrobiellen Wirkstoffe, Germizide, Fungizide, Antioxidantien oder Korrosionsinhibitoren.

6. Duftstoffkörper nach einem der vorherigen Ansprüche, wobei der Trägerstoff eine poröse oder mikroporöse Beschaffenheit aufweist.

7. Duftstoffkörper nach einem der vorherigen Ansprüche, wobei das Polyethylen ein verzweigtes Polyethylen mit einer Dichte von kleiner 0,95 g/cm³ ist.

8. Duftstoffkörper nach einem der vorherigen Ansprüche, wobei der Duftstoffkörper
95 bis 60 Gew.-%, vorzugsweise 90 bis 70 Gew.-% Trägerstoff
5 bis 40 Gew.-%, vorzugsweise 10 bis 30 Gew.-% Duftstoff,
und
0 bis 20 Gew.-%, vorzugsweise 0 bis 10 Gew.-% weitere Bestandteile
umfasst oder daraus besteht, bezogen auf das Gesamtgewicht des Duftstoffkörpers.

9. Duftstoffkörper nach einem der vorherigen Ansprüche, wobei es sich bei der Vertiefung um eine Nut, eine Nut mit ein- oder beidseitiger Hohlkehle, eine Rundnut oder einen Zinken handelt.

10. Duftstoffkörper nach einem der vorherigen Ansprüche, wobei das Polymer bzw. die Polymere nicht wasserlöslich ist bzw. sind.

11. Duftstoffkörper nach einem der vorherigen Ansprüche, wobei es sich bei dem Polymer oder bei einem, zwei, drei, mehr als drei oder sämtlichen Polymeren um einen thermoplastischen Kunststoff handelt.

12. Duftstoffkörper nach einem der vorherigen Ansprüche, wobei das Polymer oder ein, zwei, drei, mehr als drei oder sämtliche Polymer einen Schmelzpunkt von über 100 °C, vorzugsweise von über 120 °C, besonders bevorzugt von über 130 °C aufweist.

13. Verwendung eines Duftstoffkörpers nach einem der Ansprüche 1 bis 12 zur Desodorierung und Beduftung von Räumen, vorzugsweise von Innenraum von Gebäuden, Möbeln, Innenraum von Fahrzeugen oder technischen Geräten, vorzugsweise um einen Innenraum von Textilwaschmaschinen, Trocknern oder Geschirrspülmaschinen.

14. Verpackter Duftstoffkörper, bei dem ein Duftstoffkörper nach einem der Ansprüche 1 bis 11 in einer luftdichten Verpackung angeordnet ist.

15. Verfahren zur Desodorierung und Beduftung von Räumen, **dadurch gekennzeichnet, dass** ein Duftstoffkörper nach einem der Ansprüche 1 bis 12 in den Raum eingebracht wird.

## Claims

1. Fragrance body (1) for releasing fragrances, in particular in a dishwashing machine, consisting of or comprising
a) a carrier consisting of or comprising at least one polymer
and
b) at least one fragrance distributed within the carrier,
wherein the fragrance body (1) has a central axis (M) and has a continuous elongated recess (N) extending along the central axis (M) or in parallel to the central axis (M) for receiving an elongated object and wherein the recess (N) is at least partially undercut, wherein due to the elongated recess (N) the fragrance body can be placed onto elongated objects and, due to the at least partial undercutting of the recess, can remain on the elongated object without falling off,
**characterized in that** the carrier consists of a mixture of ethylene-vinyl acetate copolymer and polyethylene or comprises this mixture.

2. Fragrance body according to claim 1, wherein the fragrance body has a substantially elongated shape having a longitudinal axis, and the longitudinal axis preferably corresponds to the central axis.

3. Fragrance body according to one of the preceding claims, wherein the fragrance body additionally comprises c) fillers, preferably fillers selected from the list comprising calcium carbonate, glass fibers, glass beads, carbon fibers, natural or calcined kaolin, carbon black, silicates, amorphous or crystalline SiO₂, talcum, chalk, aluminum hydroxide, magnesium hydroxide, aluminum nitrite, aluminum silicate, barium sulfate, calcium sulfate, silica, quartz powder, aerosil, silica, clay and wollastonite.

4. Fragrance body according to one of the preceding claims, wherein the fragrance body comprises one or more recesses, preferably in the form of a trough, a slot, a notch, a blind hole or a through hole.

5. Fragrance body according to one of the preceding claims, wherein the fragrance body additionally comprises e) further additives, in particular antimicrobial agents, germicides, fungicides, antioxidants or corrosion inhibitors.

6. Fragrance body according to one of the preceding claims, wherein the carrier has a porous or microporous nature.

7. Fragrance body according to one of the preceding claims, wherein the polyethylene is a branched polyethylene having a density of less than 0.95 g/cm³.

8. Fragrance body according to one of the preceding claims, wherein the fragrance body comprises or consists of
95 to 60 wt.%, preferably 90 to 70 wt.%, carrier
5 to 40 wt.%, preferably 10 to 30 wt.%, fragrance,
and
0 to 20 wt.%, preferably 0 to 10 wt.% of further constituents,
based on the total weight of the fragrance body.

9. Fragrance body according to one of the preceding claims, wherein the recess is a groove, a groove having a fillet on one or both sides, a round groove or a prong.

10. Fragrance body according to one of the preceding claims, wherein the polymer(s) is/are not water soluble.

11. Fragrance body according to one of the preceding claims, wherein the polymer or one, two, three, more than three, or all of the polymers is a thermoplastic resin.

12. Fragrance body according to one of the preceding claims, wherein the polymer or one, two, three, more than three or all of the polymer has a melting point of above 100 °C, preferably above 120 °C, more preferably above 130 °C.

13. Use of a fragrance body according to one of the claims 1 to 12 for the deodorization and scenting of rooms, preferably interiors of buildings, furniture, interiors of vehicles or technical devices, preferably an interior of textile washing machines, dryers or dishwashing machines.

14. Packaged fragrance body, wherein a fragrance body according to one of the claims 1 to 11 is arranged in an airtight package.

15. Method for deodorizing and scenting rooms, **characterized in that** a fragrance body according to one of the claims 1 to 12 is introduced into the room.

## Revendications

1. Diffuseur de parfum (1) destiné à diffuser des parfums, en particulier dans un lave-vaisselle, comprenant, ou en étant constitué
a) un support comprenant, ou en étant constitué, au moins un polymère, et
b) au moins un parfum réparti dans le support,
le diffuseur de parfum (1) présentant un axe central (M) et un renfoncement oblong continu (N), s'étendant le long de l'axe central (M) ou parallèlement à l'axe central (M), destiné à recevoir un objet oblong, et le renfoncement (N) étant au moins partiellement contre-dépouillé,
le diffuseur de parfum étant, par le renfoncement oblong (N), fixé sur des objets oblongs, et, du fait de la contre-dépouille au moins partielle du renfoncement, pouvant rester sur l'objet oblong sans tomber, **caractérisé en ce que** le support est constitué d'un mélange d'un copolymère éthylène-acétate de vinyle et de polyéthylène ou comprend ce mélange.

2. Diffuseur de parfum selon la revendication 1, le diffuseur de parfum présentant pour l'essentiel une forme oblongue comportant un axe longitudinal, et l'axe longitudinal correspondant de préférence à l'axe central.

3. Diffuseur de parfum selon l'une des revendications précédentes, le diffuseur de parfum comprenant en outre c) des charges, de préférence des charges choisies dans la liste comprenant le carbonate de calcium, les fibres de verre, les billes de verre, les fibres de carbone, la kaolin naturel ou calciné, le noir de carbone, les silicates, le SiO₂ amorphe ou cristallin, le talc, la craie, l'hydroxyde d'aluminium, l'hydroxyde de magnésium, le nitrite d'aluminium, le silicate d'aluminium, le sulfate de baryum, le sulfate de calcium, la silice, la poudre de quartz, de l'Aerosil, des silices, de l'argile et de la wollastonite.

4. Diffuseur de parfum selon l'une des revendications précédentes, le diffuseur de parfum comportant un ou plusieurs évidements, de préférence sous forme d'une auge, d'une rainure, d'une entaille, d'un trou borgne ou d'un trou traversant.

5. Diffuseur de parfum selon l'une des revendications précédentes, le diffuseur de parfum comprenant en outre e) d'autres additifs, en particulier des matières actives antimicrobiennes, des germicides, des fongicides, des antioxydants ou des inhibiteurs de corrosion.

6. Diffuseur de parfum selon l'une des revendications précédentes, dans lequel le support présente une structure poreuse ou microporeuse.

7. Diffuseur de parfum selon l'une des revendications précédentes, dans lequel le polyéthylène est un polyéthylène ramifié ayant une masse volumique inférieure à 0,95 g/cm³.

8. Diffuseur de parfum selon l'une des revendications précédentes, le diffuseur de parfum comprenant
95 à 60 % en poids, de préférence 90 à 70 % en poids d'un support
5 à 40 % en poids, de préférence 10 à 30 % en poids d'un parfum,
et
0 à 20 % en poids, de préférence 0 à 10 % en poids d'autres constituants,
ou en est constitué, par rapport au poids total du diffuseur de parfum.

9. Diffuseur de parfum selon l'une des revendications précédentes, le renfoncement étant une gorge, une gorge comportant une cannelure à une ou deux faces, une gorge arrondie ou une entaille en queue d'aronde.

10. Diffuseur de parfum selon l'une des revendications précédentes, dans lequel le ou les polymères sont insolubles dans l'eau.

11. Diffuseur de parfum selon l'une des revendications précédentes, le polymère, ou un, deux, trois, plus de trois ou la totalité des polymères, représentent un plastique thermoplastique.

12. Diffuseur de parfum selon l'une des revendications précédentes, dans lequel le polymère ou un, deux, trois, plus de trois ou la totalité des polymères présentent un point de fusion supérieur à 100 °C, de préférence supérieur à 120 °C, d'une manière particulièrement préférée supérieur à 130 °C.

13. Utilisation d'un diffuseur de parfum selon l'une des revendications 1 à 12 pour désodoriser ou parfumer des locaux, de préférence l'espace intérieur de bâtiments, de meubles, de l'intérieur de véhicules ou d'appareils techniques, de préférence un espace intérieur de lave-linge, de sécheurs ou de lave-vaisselle.

14. Diffuseur de parfum emballé, dans lequel un diffuseur de parfum selon l'une des revendications 1 à 11 est disposé dans un emballage étanche à l'air.

15. Procédé de désodorisation et de parfumage d'espaces, **caractérisé en ce qu'**un diffuseur de parfum selon l'une des revendications 1 à 12 est placé dans l'espace.
